(19) Europäisches Patentamt
European Patent Office
Office européen des brevets

(11) Veröffentlichungsnummer: **0 001 804**
**B1**

(12) **EUROPÄISCHE PATENTSCHRIFT**

(45) Veröffentlichungstag der Patentschrift: **19.05.82**

(21) Anmeldenummer: **78101220.8**

(22) Anmeldetag: **25.10.78**

(51) Int. Cl.³: **C 07 C 43/275,**
C 07 C 43/174,
C 07 C 47/228,
C 07 C 59/64,
C 07 C 69/736,
C 07 C 69/63,
C 07 C 103/26,
C 07 C 121/38,
C 07 C 153/063,
A 01 N 37/38

(54) **Beta-Naphthyl- und Beta-Tetrahydronaphthyl-phenyläther, Verfahren zu ihrer Herstellung und ihre Verwendung als herbizide Mittel.**

(30) Priorität: **29.10.77 DE 2748658**

(43) Veröffentlichungstag der Anmeldung:
**16.05.79 Patentblatt 79/10**

(45) Bekanntmachung des Hinweises auf die Patenterteilung:
**19.05.82 Patentblatt 82/20**

(84) Benannte Vertragsstaaten:
**BE CH DE FR GB NL**

(56) Entgegenhaltungen:
**DE - A - 2 136 828**
**JP - B - 7 515 512**
**DE - A - 2 611 695**

(73) Patentinhaber: **HOECHST AKTIENGESELLSCHAFT**
**Postfach 80 03 20**
**D-6230 Frankfurt/Main 80 (DE)**

(72) Erfinder: **Hörlein, Gerhard, Dr.**
**Assmannshäuser Weg 23**
**D-6000 Frankfurt/Main 71 (DE)**
Erfinder: **Nestler, Hans Jürgen, Dr.**
**Steinweg 15**
**D-6240 Königstein/Taunus (DE)**
Erfinder: **Köcher, Helmut, Dr.**
**Kleiststrasse 9**
**D-6238 Hofheim am Taunus (DE)**
Erfinder: **Langelüddeke, Peter, Dr.**
**Nelkenweg 5**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 804 B1

β-Naphthyl- und β-Tetrahydronaphthyl-phenyläther, Verfahren zu ihrer Herstellung und ihre Verwendung als herbizide Mittel

Es ist bekannt, daß β-Naphthoxy-propionsäurederivate eine selektive herbizide Wirkung gegen breitblättrige Unkräuter besitzen und speziell zur Bekämpfung von unerwünschten Pflanzenwuchs beim Reisanbau Verwendung finden (JA—OS 125 740—76). Wie bei allen Phenoxy- und Naphthoxy-alkancarbonsäurederivaten [R. Wegler (Hrsg.): Chemie der Pflanzenschutz- und Schädlingsbekämpfungsmittel, Bd. 2 (1970), S. 276 ff.] vom Wuchsstofftypus fehlt auch bei diesen Produkten eine Wirkung gegen grasartige Unkräuter.

Gegenstand der Erfindung sind herbizide Mittel, die gekennzeichnet sind durch einen Gehalt an einer Verbindung der allgemeinen Formel I

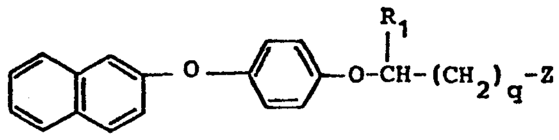

$R_1$ = Wasserstoff der $(C_1—C_4)$-Alkyl,
q = eine ganze Zahl von 0—2,

Z = eine Gruppe der Formel 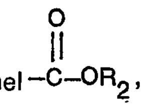, 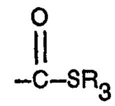

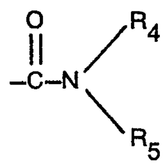, 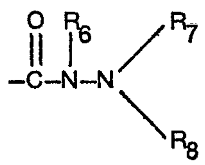, 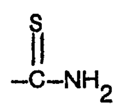,

$-CN$, $-CH_2OH$, 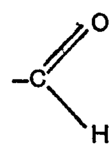, 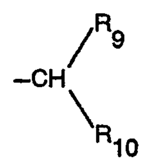,

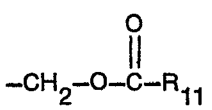, 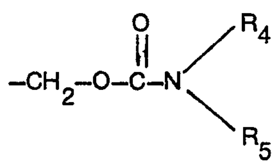 oder

$-CH_2-O-SO_2-R_{12}$,

$R_2$ = H, $(C_1—C_{12})$-Alkyl, das gegebenenfalls ein- bis sechsmal durch Halogen und/oder durch Hydroxy, $(C_1—C_6)$-Alkoxy, $(C_1—C_4)$-Alkylthio, $(C_1—C_6)$-Alkoxy$(C_2—C_6)$-alkoxy, Halogen-$(C_1—C_2)$-alkoxy, Methoxy-äthoxyäthoxy, $(C_1—C_4)$-Alkylamino, Di-$(C_1—C_4)$-alkylamino, Phenyl, Oxiranyl oder Phenoxy substituiert ist, wobei letzteres ebenfalls ein- bis zweifach durch Halogen und/oder $(C_1—C_4)$-Alkyl substituiert sein kann; $(C_5—C_6)$-Cycloalkyl, Halogen-$(C_5—C_6)$-alkyl, $(C_3—C_6)$-Alkenyl, Halogen-$(C_3—C_6)$-alkenyl, $(C_5—C_6)$-Cyclqalkenyl, $(C_3—C_4)$-Alkinyl, das gegebenenfalls einoder zweifach durch $(C_1—C_6)$-Alkyl, Phenyl, Halogen und/oder $(C_1—C_2)$-Alkoxy substituiert ist; Phenyl, das gegebenenfalls ein- bis dreifach durch $(C_1—C_4)$-Alkyl, $(C_1—C_4)$-Alkoxy, Halogen, $NO_2$ und/oder $CF_3$ substituiert ist, Furfuryl, Tetrahydrofurfuryl oder ein Kationäquivalent einer organischen oder anorganischen Base ist;

$R_3$ = $(C_1—C_6)$-Alkyl, das gegebenenfalls durch $(C_1—C_4)$-Alkoxy, Halogen oder ebenfalls ein- bis dreifach durch $(C_1—C_4)$-Alkyl und/oder Halogen substituiertes Phenyl substituiert ist, $(C_3—C_6)$-Alkenyl oder Phenyl, das gegebenenfalls ein- bis dreifach durch $(C_1—C_4)$-Alkyl und/oder Halogen substituiert ist, bedeutet,

$R_4$ und $R_5$ gleich oder verschieden sind und H, ($C_1$—$C_4$)-Alkyl, Hydroxy-($C_1$—$C_6$)-alkyl, ($C_5$—$C_6$)-Cycloalkyl oder Phenyl, das gegebenenfalls ein- bis dreifach durch ($C_1$—$C_4$)-Alkyl, ($C_1$—$C_4$)-Alkoxy, Halogen und/oder $CF_3$ substituiert ist, mit der Maßgabe, daß $R_4$ und $R_5$ nicht gemeinsam Phenyl sind, bedeuten oder gemeinsam eine Methylenkette mit 2, 4 oder 5 Gliedern bilden, in der eine $CH_2$-Gruppe gegebenenfalls durch O, NH oder $N(CH_3)$ ersetzt sein kann,

$R_6$ = H oder $CH_3$ ist,

$R_7$ = H, $CH_3$ oder $C_2H_5$ ist,

$R_8$ = H, $CH_3$, $C_2H_5$ oder Phenyl ist,

$R_9$ und $R_{10}$ gleich oder verschieden sind und ($C_1$—$C_4$)-Alkoxy, ($C_1$—$C_4$)-Alkylthio, ($C_1$—$C_4$)-Alkylamino oder Di-($C_1$—$C_4$)-alkylamino bedeuten oder zusammen einen Rest der Formel =N—$R_{12}$ darstellen,

$R_{11}$ = ($C_1$—$C_6$)-Alkyl, ($C_1$—$C_6$)-Halogenalkyl, $C_3$-, $C_5$- oder $C_6$-Cycloalkyl, ($C_3$—$C_6$)-Alkenyl, Phenyl, ($C_1$—$C_4$)-Alkylphenyl, ($C_1$—$C_4$)-Alkoxyphenyl, Halogenphenyl, Trifluormethylphenyl, Nitrophenyl oder einen Rest der Formel

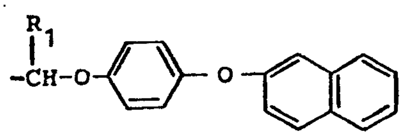

darstellt, sowie

$R_{12}$ = ($C_1$—$C_4$)-Alkyl oder Phenyl, das gegebenenfalls ein- bis dreifach durch Halogen, $CF_3$, $NO_2$ und/oder ($C_1$—$C_4$)-Alkyl substituiert ist, bedeutet.

Die in den Resten $R_1$—$R_5$, $R_{11}$ und $R_{12}$ aufgeführten Alkyl-, Alkenyl- und Alkinylreste können sowohl geradkettig wie auch verzweigt sein. "Halogen" steht bevorzugt für Chlor oder Brom.Bevorzugt als Z sind die Reste —$COOR_2$, —$CH_2OH$ und —$CH_2O$—$COR_{11}$ (q=0), in denen weiderum $R_2$ vorzugsweise Alkyl oder Alkoxyalkyl bzw. $R_{11}$ Alkyl bedeuten. Die Verbindungen der Formel I sind größtenteils neu, lediglich die 2-[4-(2-Naphthoxy)-phenoxy]-propionsäure ist aus der DE—OS 2 136 828 als Verbindung mit lipidsenkenden Eigenschaften bekannt. Gegenstand der Erfindung sind daher auch Verbindungen der Formel I mit Ausnahme solcher, in denen $R_2$ Wasserstoff bedeuten.

Die Verbindungen der Formel I lassen sich an sich bekannten bzw. nach bekannten Verfahren hergestellten Ausgangsmaterialien synthetisieren, indem man Verbindungen der Formel II

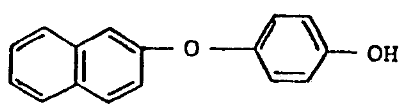

II

oder deren Alkalisalze mit Verbindungen der Formel

III 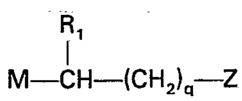 oder 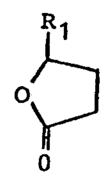 IV

in denen $R_1$ und Z obengenannte Bedeutungen haben und M für Chlor, Brom, die Mesyloxy- oder eine im aromatischen Ring gegebenenfalls substituierten Benzolsulfonyloxygruppe steht, umsetzt und gewünschtenfalls die erhaltenen Verbindungen der Formel I durch Verseifung, Veresterung, Umesterung, Salzbildung, Reduktion, Amidierung, Acylierung, Dehydratisierung, Sulfhydrierung, Acetalisierung oder Oximbildung in entsprechende Derivate der Formel I überführt.

Die Umsetzung von II mit III kann in protischen oder aprotischen Lösungsmitteln (z. B. Wasser, Dimethylformamid, Dimethylsulfoxid, Sulfolan, N-Methylpyrrolidon, Hexamethylphosphorsäuretriamid, Acetonitril, Aceton, Methyläthylketon, Benzol, Toluol, Xylol, Chlorbenzol, o-Dichlorbenzol) durchgeführt werden, wobei entweder die Salze der β-Naphthoxyphenole eingesetzt werden, oder bei Verwendung der freien Phenole durch Zusatz von säurebindenden Mitteln für das Abfangen der entstehenden Säuren gesorgt wird. Die erforderlichen Reaktionstemperaturen und Reaktionszeiten werden weitgehend durch die zum Einsatz gekommenen Lösungsmittel bestimmt.

Bei Umsetzung der freien β-Naphthoxy-phenole mit Halogenalkancarbonsäurederivaten können als Reaktionsmedien aprotische dipolare Lösungsmittel wie Aceton, Methyläthylketon oder Acetonitril eingesetzt werden. Dabei erweist sich die Verwendung von Alkalicarbonaten insbesondere Natrium- oder Kaliumcarbonat, in äquimolaren Mengen oder einem geringfügigen Überschuß, als vorteilhaft. Die Reaktion wird zweckmäßigerweise bei der Siedetemperatur des jeweiligen Lösungsmittels vorgenommen, die erforderlichen Reaktionszeiten liegen zwischen ca. 3 Stunden und 30 Stunden. Die Struktur des Phenols beeinflußt die Reaktionszeit und die Auswahl des Lösungsmittels in erheblichem Maße.

Die beschriebene Reaktionsdurchführung wird auch bei Einsatz von Sulfonsäureestern, z. B. den Mesylaten oder Tosylaten als Verätherungskomponente — insbesondere bei Herstellung optisch aktiver Präparate — bevorzugt. In Aceton kann dabei die Reaktionsdauer bis zu 70 Stunden betragen.

Bei Verwendung höher siedender aprotischer dipolarer Lösungsmittel wie Dimethylformamid, Dimethylacetamid, Dimethylsulfoxid, Sulfolan, N-Methylpyrrolidon oder Hexamethylphosphosäuretriamid werden zweckmäßig Temperaturen unterhalb der jeweiligen Siedetemperatur gewählt, da diese Lösungsmittel — insbesondere in Gegenwart von Basen — bei Normaldruck zum Teil bereits unter erheblicher Zersetzung sieden. Man wählt vorzugsweise Temperaturen zwischen 90° und 160°C, die Reaktionszeiten betragen dabei oft nur wenige Minuten bis ca. 2—3 Stunden. Als Hilfsbasen — in vorzugsweise äquimolaren Verhältnissen eingesetzt — sind hierbei außer den Alkalicarbonaten auch Alkali- und Erdalkalihydroxide, insbesondere Natrium- und Kalium- hydroxid, geeignet.

Bei Einsatz der Phenole in Form ihrer Salz, z.B. als Natrium- oder Kaliumphenolate, eignen sich aromatische Lösungsmittel wie Benzol, Toluol oder Xylol sowie chlorierte Aromaten wie Chlorbenzol oder o-Dichlorbenzol als Reaktionsmedien. Bei Temperaturen von 80°—160°C werden im allgemeinen Reaktionszeiten zwischen 1 und 6 Stunden benötigt. Die Verwendung einer Base ist in diesen Fällen nicht erforderlich.

Nach den genannten Methoden ist bei Verwendung der entsprechenden Synthesekomponenten der Formel III die größte Zahl der erfindungsgemäßen $\beta$-Naphthyl-phenyläther in guten Ausbeuten direkt zugänglich. In besonderen Fällen ist es erforderlich oder von Vorteil, die gewünschten Derivate durch Sekundärumwandlung von in erster Stufe erhaltenen Verbindungen herzustellen. Diese Sekundärumwandlungen erfolgen mit Hilfe literaturbekannter Verfahren. So werden freie Säuren der Formel I (Z = —COOH) nachträglich in Aldehyde (Z = —CHO), Ester (Z = $COOR_2$)- Salze (Z = —COOKat),

Amide 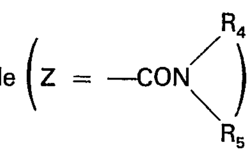, Hydrazide 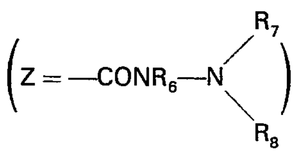 oder

Thiolester (Z = $—COSR_3$) überführt; hierzu bildet man z. B. aus den freien Säuren zunächst die Säurehalogenide und reduziert diese mit katalytisch erregtem Wasserstoff nach Rosenmund (→ Aldehyde) oder setzt die Säurehalogenide mit Alkoholen (→ Ester), Aminen (→ Amide), Hydrazinen (→ Hydrazide) oder Mercaptanen (→ Thiolester) um. Die nach dem Direktverfahren gut zugänglichen Säureester (Z = $—COOR_2$) können gewünschtenfalls verseift und die erhaltenen Säuren in der obigen Weise in andere funktionelle Derivate überführt werden. Nitrile (Z = —CN) können durch Dehydratisierung von Amiden (Z = $—CONH_2$) mittels $POCl_3$ oder $P_2O_5$ erhalten und ihrerseits mit $H_2S$ in Thioamide (Z = $—CSNH_2$) umgewandelt werden. Aus Estern kann man durch direkte Umsetzung mit Aminen oder Hydrazinen die Amide bzw. Hydrazide erhalten. Reduktion der Ester mit Metallhydriden, z. B. $LiAlH_4$, liefert Alkohole (Z = $—CH_2OH$), die wiederum mit entsprechenden Acylierungsmitteln in Carbonsäureester (Z = $—CH_2—O—COR_{11}$), Carbamidsäureester

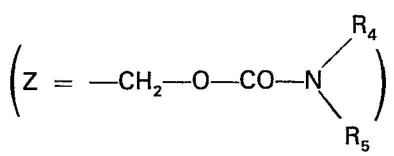

oder Sulfonsäureester (Z = $—CH_2—OSO_2R_{12}$) umgewandelt werden können. Schließlich lassen sich aus den Aldehyden (Z = —CHO) durch Umsetzung mit Alkoholen, Mercaptanen, Mercaptanen, primären oder sekundären Aminen deren Acetale, Mercaptale, Schiff'sche Basen bzw. Aminale herstellen

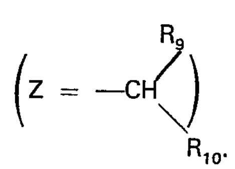

Die $\beta$-Naphthyl-phenyläther der Formel I sind in allen Fällen, in denen $R_1 \neq H$ ist, Racemate, d.h. optisch inaktive Gemische zweier enantiomerer (= optisch aktiver) Komponenten mit entgegengesetztem Drehsinn, wobei das zwischen O und $(CH_2)_q$ befindliche Kohlenstoffatom das Zentrum der Chiralität darstellt. Überraschenderweise wurde gefunden, daß die D-Entantiomeren in besonderer Weise herbizid wirksam sind. Die vorliegende Erfindung betrifft daher sowohl die optisch inaktiven Racemate als auch die optisch aktiven Enantiomeren, insbesondere deren D-Form.

Man erhält die optisch aktiven Isomeren der Formel I beispielsweise, indem man als Ausgangsstoffe der Formel III optisch aktive Verbindungen einsetzt, deren chirales Zentrum dem Rest M benachbart ist.

Die enantiomeren Formen der Verbindungen nach Formel I sind ferner durch Spaltung der Racemate in ihre Antipoden unter Verwendung von optisch aktiven Hilfsstoffen zugänglich. Zur Ausführung dieser Operation werden die Derivate in eine zur Trennung geeigneten Form, in den meisten Fällen in die freien Säuren überführt. Nachträgliche Rückführung in das Ausgangsderivat oder andere gewünschte Derivate ist nach bekannten chemischen Verfahren möglich.

Die erfindungsgemäßen Mittel zeichnen sich durch eine gute herbizide Wirkung gegen Ungräser im Vor- und Nach-auflauf aus. Von den aus den DT—OS 22 23 894 und 26 01 548 bekannten Phenoxy-phenoxy-alkancarbonsäurederivaten unterscheiden sich die neuen Verbindungen durch eine zusätzliche, deutlich ausgeprägte Wirkung gegen zweikeimblättrige Unkräuter.

Auch in hohen Dosierungen sind die Mittel verträglich gegenüber vielen wichtigen Kulturpflanzen. Sie eignen sich daher als selektive Herbizide in landwirtschaftlichen und gärtnerischen Kulturen. Sie können mit anderen Herbiziden, Insektiziden und Fungiziden kombiniert werden.

Die Mittel enthalten die Wirkstoffe der Formel I im allgemeinen zu 2—95 Gew.-%. Sie können als benetzbare Pulver, emulgierbare Konzentrate, versprühbare Lösungen, Stäubemittel oder Granulate in den üblichen Zubereitungen angewendet werden.

Benentzbare Pulver sind in Wasser gleichmäßig dispergierbare Präparate, die neben dem Wirkstoff außer einem Verdünnungsoder Inertstoff noch Netzmittel, z. B. polyoxäthylierte Alkylphenole, polyoxäthylierte Oleyl-, Stearylamine, Alkyl- oder Alkylphenyl-sulfonate und Dispergiermittel, z. B. ligninsulfsaures Natrium, 2,2′-dinaphthylmethan-6,6′-disulfonsaures Natrium oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten und Zusatz eines nichtionischen Netzmittels, beispielsweise eines polyoxäthylierten Alkylphenols oder eines polyoxäthylierten Oleyl- oder Stearylamins, erhalten.

Stäubemittel erhält man durch Vermahlen des Wirkstoffes mit fein verteilten, festen Stoffen, z. B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium, oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

Bei den herbiziden Mitteln können die Konzentrationen der Wirkstoffe in den üblichen Formulierungen verschieden sein. In benetzbaren Pulvern variiert die Wirkstoffkonzentration z. B. zwischen etwa 10% und 95%, der Rest besteht aus den oben angegebenen Formulierungszusätzen. Bei emulgierbaren Konzentraten ist die Wirkstoffkonzentration etwa 10% bis 80%. Staubförmige Formulierungen enthalten meistens 5 bis 20% an Wirkstoff, versprühbare Lösungen etwa 2 bis 20%. Bei Granulaten hängt der Wirkstoffgehalt z. T. davon ab, ob die wirksame Verbindung flüssig oder fest vorliegt und welche Granulierhilfsmittel, Füllstoffe usw. verwendet werden.

Zur Anwendung werden die handelsüblichen Konzentrate gegebenenfalls in üblicher Weise verdünnt, z. B. bei benetzbaren Pulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u.a. variiert die erforderliche Aufwandmenge. Sie kann innerhalb weiter Grenzen schwanken, z.B. zwischen 0,05 und 10,0 kg/ha oder mehr Aktivsubstanz, vorzugsweise liegt sie jedoch zwischen 0,1 und 5 kg/ha.

## Herstellungsbeispiele

Die erfindungsgemäßen Verbindungen sind vielfach hochviskose, nur schwer destillierbare Öle. Zur Identifizierung sind für diese Produkte in Tabelle 2 die NMR-Daten aufgeführt. Bei Festsubstanzen ist der Schmelzpunkt angegeben.

### Beispiele 1

2-[4-(2-Naphthoxy)-phenoxy]-propionsäure-methylester

In eine bei 100°C gerührte Suspension von 25,8 g (0,1 Mol) trockenem Natrium-4-(2-naphthoxy)-phenolat in 100 ml Xylol werden in 15 Minuten 12,3 g (0,1 Mol) 2-Chlor-propionsäuremethylester eingetropft. Nach Beendigung der Zugabe steigert man die Temperatur langsam auf 140°C und erhitzt anschließend 5 Stunden zum Sieden unter Rückfluß. Nach Erkalten wird das entstandene Natriumchlorid durch Ausschütteln der Xylolphase mit Wasser abgetrennt; man entfernt das Lösungsmittel durch Abdestillieren und fraktioniert den Rückstand im Hochvakuum.

Es werden 29,6 g (92% d. Th.) 2-[4-(2-Naphthoxy)-phenoxy]-propionsäure-methylester vom Fp. 91° bis 93°C erhalten.

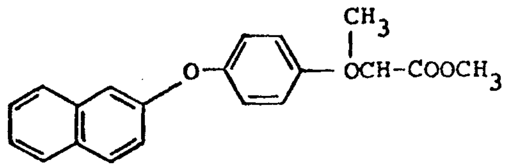

Beispiel 2

2-[4-(2-Naphthoxy)-phenoxy]-propionsäure-isobutylester

Eine Mischung von 11,8 g (0,05 Mol) 4-(2-Naphthoxy)-phenol, 10,5 g (0.05 Mol) 2-Brompropionsäure-isobutylester und 6,9 g (0,05 Mol) gepulvertem, wasserfreiem Kaliumcarbonat wird in 100 ml Aceton 18 Stunden zum Sieden unter Rückfluß erhitzt. Nach Erkalten saugt man vom entstandenen Kaliumbromid/Kaliumhydrogencarbonat-Salzgemisch ab, entfernt das Aceton durch Abdestillieren und erhält als Rückstand 16,9 g (93% d. Th.) 2-[4-(2-Naphthoxy)-phenoxy]-propionsäure-isobutylester. Durch Destillation im Hochvakuum oder durch Chromatographieren kann das Rohprodukt gereinigt werden.

NMR-Daten s. Tabelle 2.

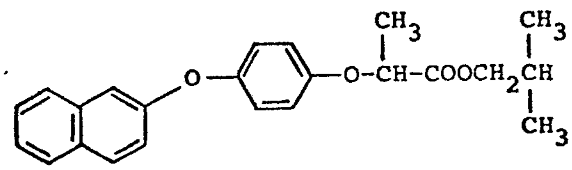

Beispiel 3

D-(+)-2-[4-(2-Naphthoxy)-phenoxy]-propionsäure-äthylester

Eine Mischung von 11,5 g (48,7 mMol) 4-(2-Naphthoxy)-phenol, 13,7 g (50,4 mMol) L-(−)-Milchsäureäthylester-tosylat uind 7,4 g (53,6 mMol) gepulvertem, wasserfreiem Kaliumcarbonat wird in 80 ml Acetonitril 25 Stunden zum Sieden unter Rückfluß erhitzt. Nach Erkalten saugt man vom entstandenen Kaliumtoluolsulfonat/Kaliumhydrogencarbonat-Salzgemisch ab, entfernt das Lösungsmittel durch Verdampfen und reinigt den rohen D-(+)-2-[4-(2-Naphthoxy)-phenoxy]-propionsäure-äthylester nach Aufnehmen in ethylenchlorid durch Chromatographieren an Kieselgel. Man erhält 12,9 g (79% d. Th.) gereinigtes Produkt mit einem Drehwert von $[\alpha]_D^{25} = 18{,}4°$ (1 m in Chloroform) als nichtkristalline Substanz.

NMR-Daten s. Tabelle 2.

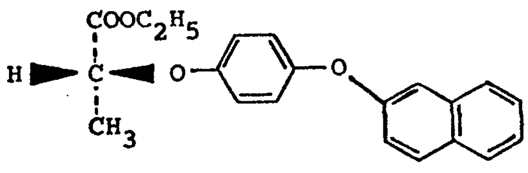

Beispiel 4

2-[4-(2-Naphthoxy)-phenoxy]-propionamid

30,8 g (0,01 Mol) 2-[4-(2-Naphthoxy)-phenoxy]-propionsäure (Beispiel 31) werden in 200 ml Benzol gelöst und nach Zugabe von 14,3 g (0,12 Mol) Thionylchlorid durch 8-stündiges Erhitzen zum Sieden unter Rückfluß in das Säurechlorid überführt. Durch Abdestillieren des überschüssigen Thionylchlorids und des Benzols erhält man 32,7 g Rohsäurechlorid. Dieses wird in 60 ml Toluol gelöst und bei Raumtemperatur zu einer ammoniakalischen Toluollösung getropft. Man rührt unter ständigem Einleiten von Ammoniak 2 Stunden nach, wobei zuletzt auf ca. 80°C erhitzt wird. Man saugt nach Erkalten den ausgeschiedenen Niederschlag ab, wäscht mit Wasser und trocknet. Man erhält 24,3 g (83% d. Th.) 2-[4-(2-Naphthoxy)-phenoxy]-propionamid vom Fp. 147 bis 148°C. Aus dem Filtrat läßt sich eine weitere Fraktion von 3,6 g (12%) des Produktes gewinnen.

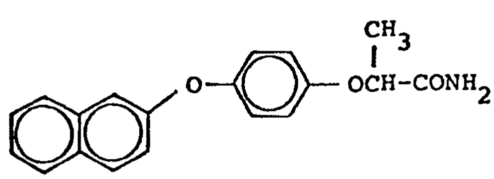

Beispiel 5

2-[4-(2-Naphthoxy)-phenoxy]-n-propanol

Eine Lösung von 32,3 g (0,1 Mol) 2-[4-(2-Naphthoxy)-phenoxy]-propionsäuremethylester (Beispiel 1) werden in 100 ml Tetrahydrofuran gelöst und innerhalb von 1,5 Stunden zu einer auf 65°C erhitzten Suspension von 3,8 g (0,1 Mol) Lithiumaluminiumchlorid in 50 ml Tetrahydrofuran getropft. Anschließend erhitzt man 1 Stunde zum Sieden unter Rückfluß kühlt danach ab und tropft zur Zersetzung des überschüssigen Lithiumaluminiumhydrids 20 ml Essigsäureäthylester, dann 60 ml Wasser zu. Nach Ansäuern mit Schwefelsäure und Abtrennen der wäßrigen Phase dampft man das Tetrahydrofuran ab, nimmt den Rückstand in Chloroform auf, wäscht mit Wasser, trocknet und entfernt das Lösungsmittel. Es verbleiben 24,8 g (84% d. Th.) 2-[4-(2-Naphthoxy)-phenoxy]-n-propanol, $n_D^{20} = 1,6173$.

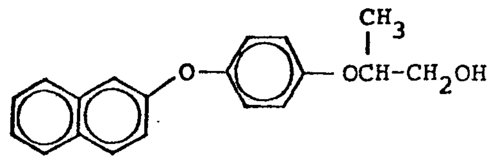

Beispiel 6

2-[4-(2-Naphthoxy)-phenoxy]-n-propyl-chloracetat

10,0 g (34 mmol) 2-[4-(2-Naphthoxy)-phenoxy]-n-propanol werden zusammen mit 4,0 g (40 mmol) Triethylamin in 50 ml Toluol gelöst. Zu dieser Lösung tropft man innerhalb von 40 min bei Raumtemperatur eine Lösung von 4,2 g (37 mmol) Chloracetylchlorid. Nach beendeter Zugabe wird 6 Stunden auf 100°C erhitzt. Man saugt nach Abkühlen vom entstandenen Triethylammonium-Hydrochlorid-Niederschlag ab und wäscht das Filtrat mit 2 n Salzsäure und Wasser. Die Toluollösung wird über 50 g Kieselgel filtriert; man destilliert das Lösungsmittel unter vermindertem Druck ab und erhält 10,7 g (85% d.Th.) 2-[4-(2-Naphthoxy)-phenoxy]-n-propyl-chloracetat als nicht kristalline Substanz, $n_D^{20} = 1,5980$.

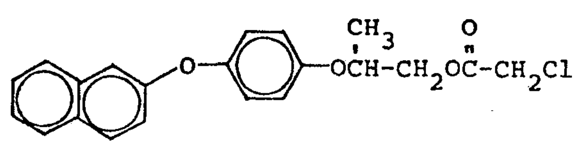

Beispiel 7

N-Methyl-O-{2-[4-(2-Naphthoxy)-phenoxy]-n-propyl}-carbamat

10,3 g (35 mmol) 2-[4-(2-Naphthoxy)-phenoxy]-n-propanol werden in 50 ml Toluol gelöst und nach Zusatz von 2 ml Triethylamin mit 2,2 g (39 mmol) N-Methylisocyanat bei Raumtemperatur versetzt. Man erhitzt innerhalb einer Stunde auf 100°C und rührt bei dieser Temperatur 6 Stunden nach. Nach Abkühlen wird mit 100 ml Toluol verdünnt und anschließend mit 2 n Salzsäure und Wasser gewaschen. Trocknen und Abdampfen des Lösungsmittels liefert 11,7 g (95% d.Th.) N-Methyl-O-{2-[4-(2-Naphthoxy)-phenoxy]-n-propyl}-carbamat als nicht kristalline Substanz. $n_D^{20} = 1,5981$.

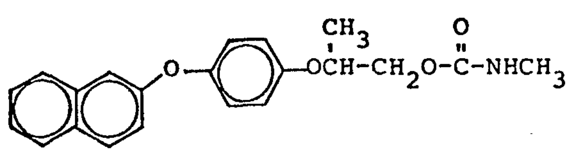

Beispiel 8

2-[4-(2-Naphthoxy)-phenoxy]-propionitril

2,0 g (6,5 mmol) 2-[4-(2-Naphthoxy)-phenoxy]-propionamid werden unter Erwärmen in 30 ml Toluol gelöst und nach Zusatz von 9,6 g (8.0 mmol) Thionylchlorid durch 20-ständiges Erhitzen zum Sieden unter Rückfluß in das Nitril überführt. Man destilliert das nicht umgesetzte Thionylchlorid und das Toluol ab, nimmt den Rückstand in 260 ml Chloroform auf und filtriert über 20 g Kieselgel. Nach Abdampfen des Chloroform verbleiben 1,8 g (96% d.Th.) 2-[4-(2-Naphthoxy)-phenoxy]-propionitril vom Fp. 93—94°C.

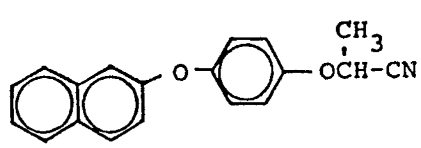

Beispiel 9

2-[4-(2-Naphthoxy)-phenoxy]-essigsäure

24,9 (79 mmol) 2-[4-(2-Naphthoxy)-phenoxy]-essigsäure-Natriumsalz (Beispiel 29) werden in 250 ml 2 n Schwefelsäure suspendiert. Durch 15 min. Erhitzen auf 90°C wird das Salz in die freie Säure umgewandelt. Man erhält durch Absaugen 23,4 g (100%) 2-[4-(2-Naphthoxy)-phenoxy]-essigsäure vom Fp. 142,5—143°C.

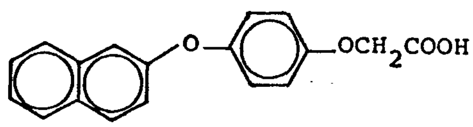

Beispiel 10

2-[4-(2-Naphthoxy)-phenoxy]-propionsäure-propargylester

13,2 g (43 mmol) 2-[4-(2-Naphthoxy)-phenoxy]-propionsäure (Beispiel 31) werden in 100 ml Benzol gelöst und nach Zugabe von 6,1 g (52 mmol) Thionylchlorid durch 8-stündiges Erhitzen zum Sieden unter Rückfluß in das Säurechlorid überführt. Durch Abdestillieren des überschüssigen Thionylchlorids und des Benzols erhält man 14,0 g Roh-Säurechlorid. Dieses wird in 30 ml Toluol gelöst und bei Raumtemperatur in 30 Minuten zu einer Lösung von 2,9 g (52 mmol) Propargylalkohol und 6,5 g (65 mmol) Triethylamin in 30 ml Toluol getropft. Man erhitzt 2,5 Stunden zum Sieden unter Rückfluß, saugt nach Abkühlen vom Niederschlag ab und wäscht das Filtrat mit Wasser. Nach Trocknen und Abdampfen des Lösungsmittels erhält man 12,8 g (86% d.Th.) 2-[4-(2-Naphthoxy)-phenoxy]-propionsäure-propargylester, $n_D^{20} = 1,5915$. Eventuell vorhandene Spuren der freien Säure werden durch Filtrieren einer Lösung des Esters in Chloroform über Kieselgel beseitigt.

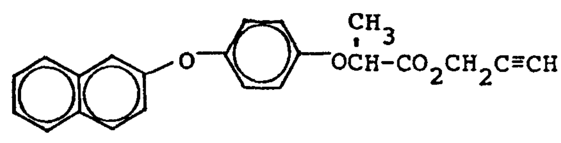

Beispiel 11

4-[4-(2-Naphthoxy)-phenoxy]-n-buttersäure

Man stelle durch Erhitzen von 23,6 g (0,10 mol) 4-(2-Naphthoxy)-phenyl mit 50 ml 2 n Natronlauge (0,10 mol) in 100 ml Dibutyl-diglykoläther auf 120°C das Natriumsalz des 4-(2-Naphthoxy)-phenols her; anschließend wird das vorhandene Wasser abdestilliert.

Man gibt 9,5 g (0,11 mol) $\delta$-Butyrolacton hinzu und erhitzt 6 Stunden auf 150°C. Der Dibutyldiglykoläther wird unter vermindertem Druck abdestilliert und der Rückstand in Wasser unter Erwärmen aufgenommen. Durch Ansäuern erhält man die 4-[4-(2-Naphthoxy)-phenoxy]-n-buttersäure.

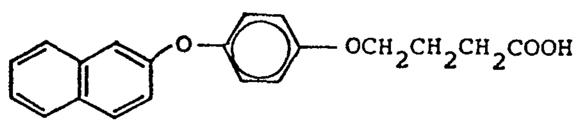

Beispiel 12

4-[4-(2-Naphthoxy)-phenoxy]-n-valeriansäure

Entsprechend der Vorschrift von Biespiel 11 erhält man die Verbindungen unter Verwendung von 11,0 g (0,11 mol) $\delta$-Valerolacton durch 6-stündiges Erhitzen auf 180°C.

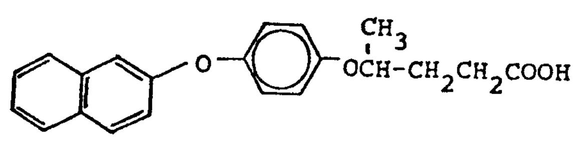

In analoger Weise erhält man unter Verwendung entsprechender Ausgangsstoffe die folgenden Verbindungen:

TABELLE 1

| Beispiel Nr. | (R) | $R_1$ | $-(CH_2)_q-Z$ | Phys. Daten | hergestellt nach Beispiel |
|---|---|---|---|---|---|
| 13 | 2-Naphthyl | H | $COOC_2H_5$ | s. Tabelle 2 | 2 |
| 14 | 2-Naphthyl | H | $CH_2CH_2COOC_2H_5$ | | 2 |
| 15 | 2-Naphthyl | $CH_3$ | $COOC_2H_5$ | s. Tabelle 2 | 2 |
| 16 | 2-Naphthyl | $CH_3$ | COO-n-$C_3H_7$ | s. Tabelle 2 | 2 |
| 17 | 2-Naphthyl | $CH_3$ | COO-i-$C_3H_7$ | s. Tabelle 2 | 2 |
| 18 | 2-Naphthyl | $CH_3$ | $COOCH_2CH_2Cl$ | | 2 |
| 19 | 2-Naphthyl | $CH_3$ | $COOCH_2CH_2CH(OCH_3)CH_3$ | | 2 |
| 20 | 2-Naphthyl | $CH_3$ | $COOCH_2$ 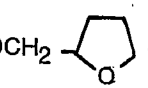 | | 2 |
| 21 | 2-Naphthyl | $CH_3$ | $CH_2CH_2COOCH_3$ | | 2 |
| 22 | 2-Naphthyl | $CH_3$ | $CONH_2$ | | 6 |
| 23 | 2-Naphthyl | $CH_3$ | $CON(CH_3)_2$ | | 6 |
| 24 | 2-Naphthyl | $CH_3$ | $CON(C_2H_5)_2$ | | 6 |
| 25 | 2-Naphthyl | $CH_3$ | CHO | | |
| 26 | 2-Naphthyl | $CH_3$ | CS–$NH_2$ | | |
| 27 | 2-Naphthyl | $CH_3$ | $COONH_4$ | | 12 |
| 28 | 2-Naphthyl | $CH(CH_3)_2$ | COO-i-$C_3H_7$ | | 2 |

TABELLE 1: (Fortsetzung)

| Beispiel Nr. | (R) | $R_1$ | $-(CH_2)_q-Z$ | Phys. Daten | hergestellt nach Beispiel |
|---|---|---|---|---|---|
| 29 | 2-Naphthyl | H | COONa | Fp. > 250° (Zers.) | 12 |
| 30 | ,, | H | CO– 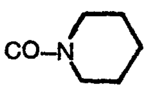 | Fp. 62–67° | 6 |
| 31 | ,, | $CH_3$ | COOH | Fp. 138° | 5 |
| 32 | ,, | $CH_3$ | COO $CH_2CHClCH_2Cl$ | $n_D^{20}$ : 1,5968 | 11 |
| 33 | ,, | $CH_3$ | $CH_2CH_2OCOCCl_3$ | $n_D^{20}$ : 1,5938 | 8 |
| 34 | ,, | $CH_3$ | $CH_2CH_2OCOC_6H_4Cl$ (p) | Fp. 75–79° | 8 |

TABELLE 2: 60 MHz-$^1$H-NMR-Daten zur Charakterisierung einzelner Verbindungen aus der Reihe der Herstellungsbeispiele

Chemische Verschiebung $\delta$ [ppm], relativ zu TMS als interner Standard, in $CDCl_3$
(in Klammern Multiplizität und relative Intensität der Signale)

| Beispiel Nr. | $-CH_3$ | $-CH_2-$ | $-\overset{\vert}{C}H$ | $-OCH_3$ | $-OCH_2-$ | $-O\overset{\vert}{C}H-$ | $H_{aromat.}$ |
|---|---|---|---|---|---|---|---|
| 2 | 0,85 (d, 6)<br>1,65 (d, 3) | | 1,90 (sept., 1) | | 3,95 (d, 2) | 4,90 (q, 1) | 6,95–7,90 (m, 11) |
| 3 | | | | | | | |
| 4 | 1,25 (t, 3)<br>1,65 (d, 2) | | | | 4,25 (q, 2) | 4,75 (q, 1) | 6,75–7,95 (m, 11) |
| 13 | 1,30 (t, 3) | | | | 4,25 (q, 2)<br>4,55 (s, 2) | | 6,80–7,95 (m, 11) |
| 15 | 1,30 (t, 3)<br>1,70 (d, 3) | | | | 4,25 (q, 2) | 4,80 (q, 1) | 6,80–8,05 (m, 11) |
| 16 | 0,95 (t, 3)<br>1,75 (d, 3) | ~1,75 (q, 2) | | | 4,20 (t, 2) | 4,80 (q, 1) | 6,80–8,05 (m, 11) |
| 17 | 1,10–1,45 (2d, 6)<br>1,70 (d, 3) | | | | | 4,70 (q, 1) | 6,80–8,05 (m, 11) |

## FORMULIERUNGSBEISPIELE

### Beispiel A

Ein emulgierbares Konzentrat wird erhalten aus
15 Gew.-Teilen Wirkstoff
75 Gew.-Teilen Cyclohexanon als Lösungsmittel
und 10 Gew.-Teilen oxäthyliertem Nonylphenol (10 AEO) als Emulgator

### Beispiel B

Ein in Wasser leicht dispergierbares benetzbares Pulver wird erhalten, indem man
25 Gew.-Teile Wirkstoff
64 Gew.-Teile kaolinhaltigen Quarz als Inertstoff
10 Gew.-Teile ligninsulfonsaures Kalium
und 1 Gew.-Teil oleylmethyltaurinsaures Natrium als Netz- und Dispergiermittel
mischt und in einer Stiftmühle mahlt.

### Beispiel C

Ein Stäubemittel wird erhalten, indem man
10 Gew.-Teile Wirkstoff
und 90 Gew.-Teile Talkum als Inertstoff
mischt und in einer Schlagmühle zerkleinert.

### Beispiel D

Ein Granulat besteht z.B. aus etwa
2—15 Gew.-Teilen Wirkstoff
98—85 Gew.-Teilen inerten Granulatmaterialien, wie z.B. Attapulgit, Bimsstein und Quarzsand.

## BIOLOGISCHE BEISPIELE

### Beispiel I

Erfindungsgemäße Verbindungen*) wurden nach dem Aussäen von Unkräutern auf Blumentöpfe gesprüht und anschließend hinsichtlich ihrer herbiziden Effektivität beobachtet und visuell bonitiert. Die Bonitierung erfolgte nach dem Schema von BOLLE (Nachrichtenblatt des Deutschen Pflanzenschutzdienstes *16*, 1964, 92—94):

*) in Form wässriger Dispersionen von Spritzpulverkonzentraten

| Wertzahl | Schadwirkung in % an | |
|---|---|---|
| | Unkräutern | Kulturpflanzen |
| 1 | 100 | 0 |
| 2 | 97,5 bis < 100 | 0 bis > 2,5 |
| 3 | 95 bis < 97,5 | 2,5 bis > 5 |
| 4 | 90 bis < 95 | 5 bis > 10 |
| 5 | 85 bis < 90 | 10 bis > 15 |
| 6 | 75 bis < 85 | 15 bis > 25 |
| 7 | 65 bis < 75 | 25 bis > 35 |
| 8 | 32,5 bis < 65 | 35 bis > 67,5 |
| 9 | 0 bis < 32,5 | 67,5 bis > 100 |

Die Ergebnisse in Tabelle Ia zeigen, daß die Substanzen sehr gute herbizide Eigenschaften haben und zahlreiche Unkräuter gut bekämpfen.

Ebenso wurden viele Kulturpflanzen in Töpfe ausgesät und vor dem Auflaufen mit den erfindungsgemäßen Verbindungen behandelt. Es zeigte sich, daß selbst hohe Dosierungen von 2,4 kg AS/ha keine oder nur geringfügige Schäden an den Kulturpflanzen verursachen. Die Bonitur erfolgte ca. 4 Wochen nach Application der Verbindungen. Die Ergebnisse sind in Tabelle Ib aufgeführt. Die Versuchstöpfe wurden unter Gewächshausbedingungen gehalten.

Beispiel II

Verschiedene Unkräuter und Kulturpflanzen wurden in Töpfe ausgesät und im Gewächshaus ca. 3 Wochen bis zur Größe von 15 bis 25 cm herangezogen. Anschließend wurden die Pflanzen im Nachauflaufverfahren mit den erfindungsgemäßen Verbindungen behandelt. Die visuelle Bonitur ca. 4 Wochen nach Applikation durchgeführt, zeigte, daß Unkräuter von den Verbindungen gut bekämpft d.h. abgetötet wurden, während viele Kulturpflanzen nicht oder ganz unbedeutend geschädigt waren (s. Tabelle II).

TABELLE Ia: Herbizide Vorauflaufwirkung gegen Ungräser und Unkräuter

| Verbindung gemäß Beispiel | Dosis kg AS /ha | ALM | SAL | LOM | ECG | AMR |
|---|---|---|---|---|---|---|
| 1 | 2,4 | 1 | 1 | 1 | 1 | – |
| | 0,6 | 2 | 2 | 1 | 1 | – |
| 15 | 2,4 | 1 | 1 | 1 | 1 | 2 |
| | 0,6 | 2 | 3 | 1 | 2 | 3 |
| 16 | 2,4 | 1 | 1 | 1 | 1 | – |
| | 0,6 | 2 | 2 | 1 | 1 | – |
| 17 | 2,4 | 1 | 1 | 1 | 1 | 1 |
| | 0,6 | 1 | 2 | 2 | 1 | 3 |

ALM = Alopecurus myosuroides

SAL = Setaria lutescens

LOM = Lolium multiflorum

ECG = Echinochloa crus galli

AMR = Amaranthus retroflexus

AS = Aktivsubstanz

TABELLE Ib: Toleranz wichtiger Kulturpflanzen im Vorauflaufverfahren

| Verbindung gemäß Beispiel | Dosis kg AS/ha | Erdnuß | Sojabohne | Bohne | Ackerbohne | Tabak | Weizen | Luzerne | Raps |
|---|---|---|---|---|---|---|---|---|---|
| 1 | 2,4 | 2 | 3 | 3 | 1 | 4 | 2 | 3 | 4 |
| 15 | 2,4 | 1 | 3 | 2 | 1 | 1 | — | 2 | 1 |
| 16 | 2,4 | 1 | 3 | 3 | 2 | 2 | 5 | 4 | 2 |

TABELLE II: Herbizide Wirkung im Nachauflaufverfahren gegen verschiedene Unkräuter und Kulturpflanzen

| Verbindung gemäß Beispiel | Dosis kg AS/ha | ALM | SAL | LOM | ECG | Weizen | Zuckerrübe | Sojabohne | Luzerne |
|---|---|---|---|---|---|---|---|---|---|
| 1 | 2,4 | 1 | – | 1 | 1 | 1 | 4 | 4 | 1 |
| | 0,6 | 4 | – | 3 | 2 | | | | |
| 15 | 2,4 | 1 | 2 | 1 | 2 | 6 | 5 | 1 | 1 |
| | 0,6 | 2 | 5 | 1 | 2 | | | | |
| 16 | 2,4 | 1 | 2 | 1 | 1 | 5 | 3 | 1 | 1 |
| | 0,6 | 2 | 3 | 1 | 1 | | | | |
| 17 | 2,4 | 1 | 1 | 1 | 1 | 2 | 2 | 1 | 1 |

ALM = Alopecurus myosuroides

SAL = Setaria lutescens

LOM = Lolium multiflorum

ECG = Echinochloa crus galli

**Patentansprüche**

1. Herbizide Mittel, gekennzeichnet durch einen Gehalt an einer Verbindung der allgemeinen Formel I

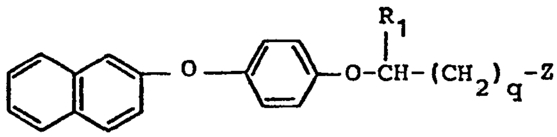

worin

$R_1$ = Wasserstoff oder ($C_1$—$C_4$)-Alkyl,

q = eine ganze Zahl von 0—2

Z = eine Gruppe der Formel 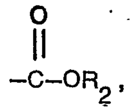, 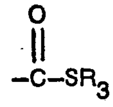

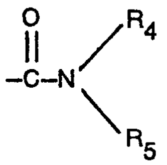, 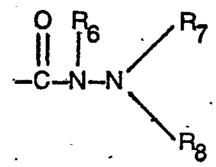, 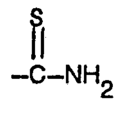,

$-CN$, $-CH_2OH$, 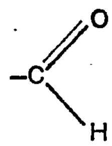, 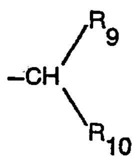,

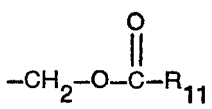, 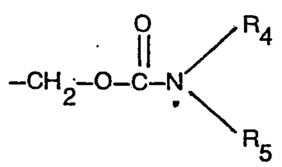 oder

$-CH_2-O-SO_2-R_{12}$,

$R_2$ = H, ($C_1$—$C_{12}$)-Alkyl, das gegebenenfalls 1 bis 6 mal durch Halogen und/oder Hydroxy, ($C_1$—$C_6$)-Alkoxy, ($C_1$—$C_4$)-Alkylthio, ($C_1$—$C_6$)-Alkoxy($C_2$—$C_6$)-alkoxy, Halogen-($C_1$—$C_2$)-alkoxy, Methoxy-äthoxy-äthoxy, ($C_1$—$C_4$)-Alkylamino, Di-($C_1$—$C_6$)-Alkylamino, Phenyl, Oxiranyl oder Phenoxy substituiert ist, wobei letzteres ebenfalls ein- bis zweifach durch Halogen und/oder ($C_1$—$C_4$)-Alkyl substituiert sein kann; ($C_5$—$C_6$)-Cycloalkyl, Halogen-($C_5$—$C_6$)-alkyl, ($C_3$—$C_6$)-Alkenyl, Halogen-($C_3$—$C_6$)-alkenyl, ($C_5$—$C_6$)-Cycloalkenyl, ($C_3$—$C_4$)-Alkinyl, das gegebenenfalls ein- oder zweifach durch ($C_1$—$C_6$)-Alkyl, Phenyl, Halogen und/oder ($C_1$—$C_2$)-Alkoxy substituiert ist, Phenyl, das gegebenenfalls ein- bis dreifach durch ($C_1$—$C_4$)-Alkyl, ($C_1$—$C_4$)-Alkoxy, Halogen, $NO_2$ und/oder $CF_3$ substituiert ist; Furfuryl, Tetrahydrofurfuryl oder ein Kationäquivalent einer organischen oder anorganischen Base ist;

$R_3$ = ($C_1$—$C_6$)-Alkyl, das gegebenenfalls durch ($C_1$—$C_4$)-Alkoxy, Halogen oder ebenfalls ein- bis dreifach durch ($C_1$—$C_4$)-Alkyl und/oder Halogen substituiertes Phenyl substituiert ist; ($C_3$—$C_6$)-Alkenyl oder Phenyl, das gegebenenfalls ein- bis dreifach durch ($C_1$—$C_4$)-Alkyl und/oder Halogen substituiert ist, bedeutet,

$R_4$ und $R_5$ gleich oder verschieden sind und H, ($C_1$—$C_4$)-Alkyl, Hydroxy-($C_1$—$C_6$)-alkyl, ($C_5$—$C_6$)-Cycloalkyl oder Phenyl, das gegebenenfalls ein- bis dreifach durch ($C_1$—$C_4$)-Alkyl, ($C_1$—$C_4$)-Alkoxy, Halogen und/oder $CF_3$ substituiert ist, mit der Maßgabe, daß $R_4$ und $R_5$ nicht gemeinsam Phenyl sind, bedeuten oder gemeinsam eine Methylenkette mit 2, 4 oder 5 Gliedern bilden, in der eine $CH_2$-Gruppe gegebenenfalls durch O, NH oder $N(CH_3)$ ersetzt sein kann,

$R_6$ = H oder $CH_3$ ist,

$R_7$ = H, $CH_3$ oder $C_2H_5$ ist,

$R_8$ = H, $CH_3$, $C_2H_5$ oder Phenyl ist,

$R_9$ und $R_{10}$ gleich oder verschieden sind und ($C_1$—$C_4$)-Alkoxy, ($C_1$—$C_4$)-Alkylthio, ($C_1$—$C_4$)-Alkylamino oder Di-($C_1$—$C_4$)-alkylamino oder zusammen einen Rest der Formel =N—$R_{12}$ darstellen,

$R_{11}$ = ($C_1$—$C_6$)-Alkyl, ($C_1$—$C_6$)-Halogenalkyl, $C_3$—$C_5$- oder $C_6$-Cycloalkyl, ($C_3$—$C_6$)-Alkenyl, Phenyl, ($C_1$—$C_4$)-Alkylphenyl, ($C_1$—$C_4$)-Alkoxyphenyl, Halogenphenyl, Trifluormethylphenyl, Nitrophenyl oder einen Rest der Formel

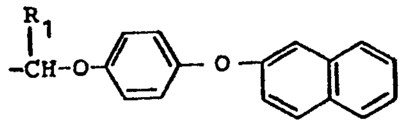

darstellt, sowie

$R_{12}$ = ($C_1$—$C_4$)-Alkyl oder Phenyl, das gegebenenfalls ein bis dreifach durch Halogen, $CF_3$, $NO_2$ und/oder ($C_1$—$C_4$)-Alkyl substituiert ist, bedeutet.

2. Herbizide Mittel gemäß Anspruch 1, gekennzeichnet durch einen Gehalt an einer Verbindung der Formel I in Form ihres D-Enantiomeren (bezogen auf das zwischen O und $(CH_2)_q$ befindliche C-Atom als Chiralitätszentrum).

3. Verbindungen der Formel I, worin $R_2$ ($C_1$—$C_{12}$)-Alkyl, das gegebenenfalls 1—6 mal durch Halogen und/oder durch Hydroxy, ($C_1$—$C_6$)-Alkoxy, ($C_1$—$C_4$)-Alkylthio, ($C_1$—$C_6$)-Alkoxy-($C_2$—$C_6$)-alkoxy, Halogen-($C_1$—$C_4$)-alkoxy, Methoxy-äthoxyäthoxy, ($C_1$—$C_4$)-Alkylamino, Di-($C_1$—$C_4$)-alkylamino, Phenyl, Oxiranyl oder Phenoxy substituiert ist, wobei letzteres ebenfalls ein- bis zweifach durch Halogen und/oder ($C_1$—$C_4$)-Alkoxy substituiert sein kann; ($C_5$—$C_6$)-Cycloalkyl, Halogen-($C_5$—$C_6$)-cycloalkyl, ($C_3$—$C_5$)-Alkenyl, Halogen-($C_3$—$C_6$)-alkenyl, ($C_5$—$C_6$)-Cycloalkenyl, ($C_3$—$C_4$)-Alkinyl, das gegebenenfalls ein- oder zweifach durch ($C_1$—$C_6$)-Alkyl, Phenyl, Halogen und/oder ($C_1$—$C_2$)-Alkoxy substituiert ist; Phenyl, das gegebenenfalls ein- bis zweifach durch ($C_1$—$C_4$)-Alkyl, ($C_1$—$C_4$)-Alkoxy, Halogen, $NO_2$ und/oder $CF_3$ substituiert ist; Furfuryl, Tetrahydrofurfuryl, oder ein Kationäquivalent einer organischen oder anorganischen Base ist, und die übrigen Reste die in Anspruch 1 angegebenen Bedeutungen haben.

4. Verbindungen der Formel I in Form ihres D-Enantiomeren (bezogen auf das zwischen O und $(CH_2)_q$ befindliche C-Atom als Chiralitätszentrum).

5. Verbindung der Formel

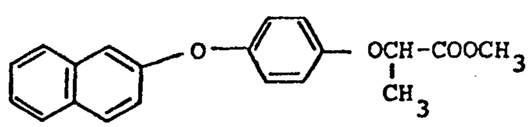

6. Verbindung der Formel

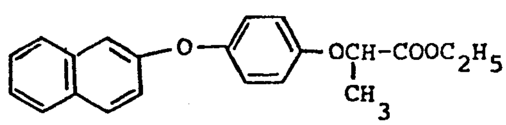

7. Verbindung der Formel

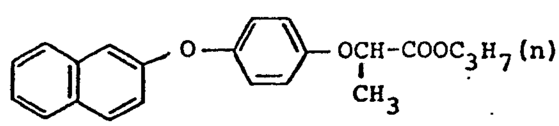

8. Verbindung der Formel

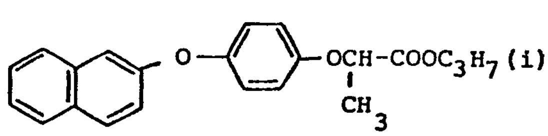

9. Verfahren zur Herstellung von Verbindungen gemäß Anspruch 3, dadurch gekennzeichnet, daß man 4-(β-Naphthoxy)-phenole der Formel II

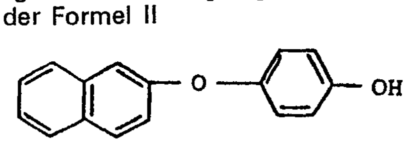

II

in Gegenwart eines säurebindenden Mittels, oder deren Alkalisalze mit Verbindungen der Formeln

III 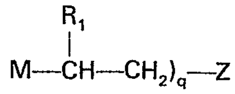 oder 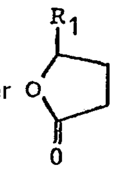 IV

in der M für Chlor, Brom, die Mesyloxy- oder eine im aromatischen Ring gegebenenfalls substituierte Benzolsulfonyloxygruppe steht, umsetzt und gewünschtenfalls die erhaltenen Verbindungen der Formel I durch Verseifung, Veresterung, Umesterung, Salzbildung, Reduktion, Amidierung, Acylierung, Dehydratisierung, Sulfhydrierung, Acetalisierung oder Oximbildung in entsprechende Derivate der Formel I überführt.

10. Verwendung von herbiziden Mitteln gemäß Anspruch 1 oder 2 zur Bekämpfung von unerwünschtem Pflanzenwuchs.

## Claims

1. Herbicidal compositions containing a compound of the formula I

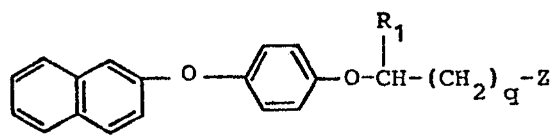

in which

$R_1$ is hydrogen or ($C_1$—$C_4$)-alkyl,

q is a whole number from zero to 2,

Z is a group of the formula 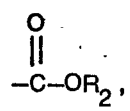, 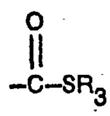

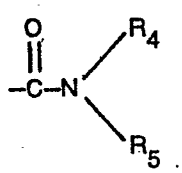, 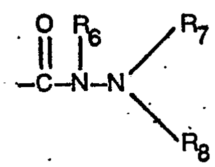, 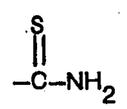,

$-CN$, $-CH_2OH$, 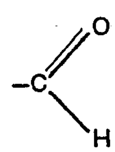, 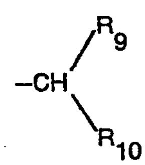,

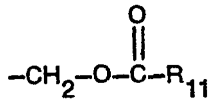, 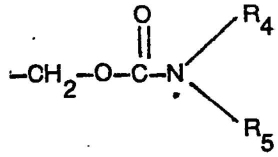 or

$-CH_2-O-SO_2-R_{12}$

$R_2$ is hydogen, ($C_1$—$C_{12}$)alkyl which may be substituted one to six times by the halogen and/or by hydroxy, ($C_1$—$C_6$)-alkoxy, ($C_1$—$C_4$)alkylthio, ($C_1$—$C_6$)alkoxy-($C_2$—$C_6$)alkoxy, halo($C_1$—$C_2$)alkoxy, methoxy-ethoxyethoxy, ($C_1$—$C_4$)alkylamino, di($C_1$—$C_4$)alkylamino, phenyl, oxiranyl, or phenoxy, (the latter optionally being substituted once or twice by halogen and/or ($C_1$—$C_4$)alkyl; ($C_5$—$C_6$)cycloalkyl, halo($C_5$—$C_6$)alkyl, ($C_3$—$C_6$)alkenyl, halo($C_3$—$C_6$)alkenyl, ($C_5$—$C_6$)cycloalkyl, ($C_3$—$C_4$)alkinyl which may

be substituted once or twice by ($C_1$—$C_6$)alkyl, phenyl, halogen and/or ($C_1$—$C_2$)alkoxy; phenyl which may be substituted one to three times by ($C_1$—$C_4$)alkyl, ($C_1$—$C_4$)alkoxy, halogen, $NO_2$ and/or $CF_3$; furfuryl, tetrahydrofurfuryl, or a cation equivalent of an organic or inorganic base;

$R_3$ is ($C_1$—$C_6$)alkyl which may be substituted by ($C_1$—$C_4$)alkoxy, halogen or phenyl (optionally mono- to tri-substituted by ($C_1$—$C_4$)alkyl and/or halogen); ($C_3$—$C_6$)alkenyl, or phenyl (optionally substituted one to three times by ($C_1$—$C_4$)alkyl and/or halogen);

$R_4$ and $R_5$, which are identical or different, are hydrogen, ($C_1$—$C_4$)alkyl, hydroxy($C_1$—$C_6$)alkyl, ($C_5$—$C_6$)cycloalkyl, or phenyl (optionally substituted one to three times by ($C_1$—$C_4$)alkyl, ($C_1$—$C_4$)alkoxy, halogen and/or $CF_3$; with the proviso that $R_4$ and $R_5$ cannot both be phenyl; or $R_4$ and $R_5$ together form a methylene chain with 2, 4 or 5 members in which one $CH_2$ group may be replaced by oxygen, NH or $N(CH_3)$;

$R_6$ is H or $CH_3$,

$R_7$ is H, $CH_3$ or $C_2H_5$,

$R_8$ is H, $CH_3$, $C_2H_5$ or phenyl,

$R_9$ and $R_{10}$, which are identical or different, are ($C_1$—$C_4$)-alkoxy, ($C_1$—$C_4$)alkylthio, ($C_1$—$C_4$)alkylamino, or di($C_1$—$C_4$)alkylamino, or together are a radical of the formula $=N—R_{12}$;

$R_{11}$ is ($C_1$—$C_6$)alkyl, ($C_1$—$C_6$)haloalkyl, $C_3$-, $C_5$- or $C_6$-cycloakyl, ($C_3$—$C_6$)alkenyl, phenyl, ($C_1$—$C_4$)alkylphenyl, ($C_1$—$C_4$)alkoxyphenyl, halophenyl, trifluoromethylphenyl, nitrophenyl or a radical of the formula

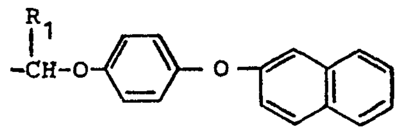

and

$R_{12}$ is ($C_1$—$C_4$)alkyl or phenyl optionally substituted one to three times by halogen, $CF_3$, $NO_2$ and/or ($C_1$—$C_4$)alkyl.

2. Herbicidal compositions as claimed in claim 1 characterized by a content of a compound of formula I in the form of its d-enantiomer (relative to the carbon atom between O and $(CH_2)_q$ as center of chirality.

3. Compounds of formula I in which $R_2$ is ($C_1$—$C_{12}$)alkyl optionally substituted one to six times by halogen, and/or hydroxy, ($C_1$—$C_6$)alkoxy, ($C_1$—$C_4$)alkylthio, ($C_1$—$C_6$)alkoxy($C_2$—$C_6$)alkoxy, halo-($C_1$—$C_4$)alkoxy, methoxyethoxyethoxy, ($C_1$—$C_4$)alkylamino, di-($C_1$—$C_4$)alkylamino, phenyl, oxiranyl or phenoxy, the latter possibly being substituted once or twice by halogen and/or ($C_1$—$C_4$)alkoxy; ($C_5$—$C_6$)cycloalkyl, halo($C_5$—$C_6$)cycloalkyl, ($C_3$—$C_5$)alkenyl, halo($C_3$—$C_6$)alkenyl, ($C_5$—$C_6$)cycloalkenyl, ($C_3$—$C_4$)alkinyl optionally substituted one to twice by ($C_1$—$C_6$)alkyl, phenyl, halogen and/or ($C_1$—$C_2$)-alkoxy; phenyl optionally substituted once or twice by ($C_1$—$C_4$)alkyl, ($C_1$—$C_4$)alkoxy, halogen, $NO_2$ and/or $CF_3$; furfuryl, tetrahydrofurfuryl, or a cation equivalent of an organic or inorganic base, and the other radicals have the meanings as defined in claim 1.

4. Compound of formula I in the form of the d-enantiomer relative to the carbon atom between O and $(CH_2)_q$ as center of chirality.

5. A compound having the formula

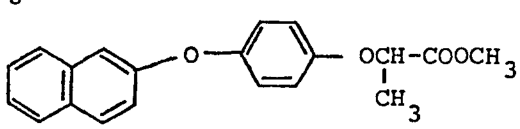

6. A compound having the formula

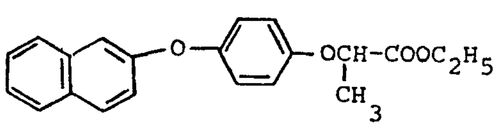

7. A compound having the formula

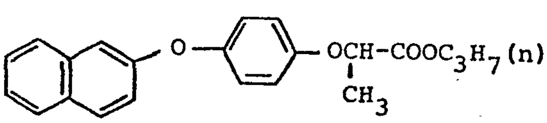

8. A compound having the formula

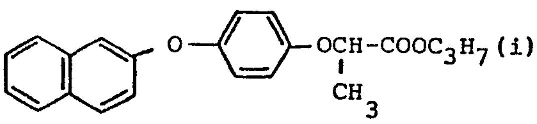

9. A process for the manufacturer of compounds as claimed in claim 3, which comprises reacting 4-(β-naphthoxy)-phenols of formula II

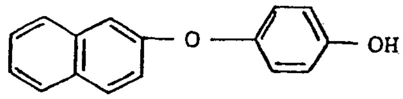

(II)

in the presence of an acid binding agent, or an alkali metal salt of said phenol, with compounds of the formulae

(III) 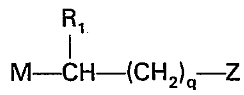 or 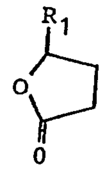 (IV)

in which M is chlorine, bromine, mesyloxy or a benzene sulfonyloxy group which may be substituted in the aromatic nucleus, and, if desired, transforming the compounds of formula I obtained into corresponding derivates of formula I by saponification, esterification, transesterification, salt formation, reduction, amidation, acylation, dehydration, sulfhydration, acetalization, or oxime formation.

10. Use of herbicidal compositions according to claims 1 or 2 for combating the growth of undesired plants.

**Revendications**

1. Produits herbicides caractérisés en ce qu'ils contiennent un composé répondant à la formule générale I

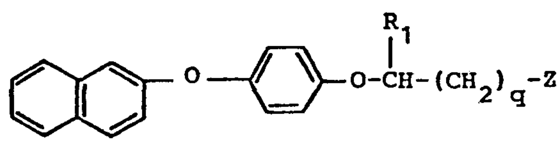

(I)

dans laquelle

$R_1$ représente l'hydrogène ou un alkyle en $C_1$—$C_4$,

q représente un nombre entier de 0 à 2,

Z représente un groupe répondant à l'une des formules suivantes:

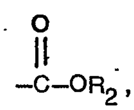 , 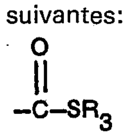

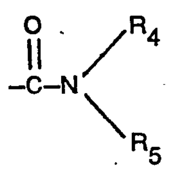 , 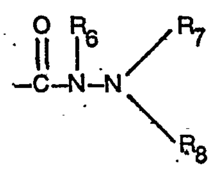 , 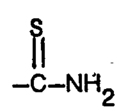 ,

$-CN$ , $-CH_2OH$ , 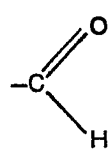 , 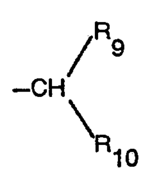

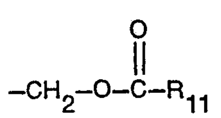 , 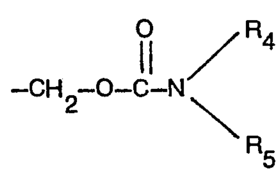 et

$-CH_2-O-SO_2-R_{12}$ ,

$R_2$ représente l'hydrogène; un alkyle en $C_1$—$C_{12}$ éventuellement porteur d'1 à 6 substituants pris dans l'ensemble constitué par les halogènes, et/ou le groupe hydroxy, les alcoxy en $C_1$—$C_6$, les alkylthio en $C_1$—$C_4$, les ($C_1$—$C_6$)alcoxy-($C_2$—$C_6$)alcoxy, les halogéno-alcoxy en $C_1$ et $C_2$, le radical méthoxy-éthoxy-éthoxy, les alkylamino en $C_1$—$C_4$, les di-($C_1$—$C_4$)alkylamino, le phényle, l'oxirannyle et le phénoxy, ce dernier pouvant également porter 1 ou 2 substituants pris dans l'ensemble constitué par les halogènes et/ou les alkyles en $C_1$—$C_4$; un cyclo-alkyle en $C_5$ ou $C_6$, un halogéno-alkyle en $C_5$ ou $C_6$, un alcényle en $C_3$—$C_6$, un halogéno-alcényle en $C_3$—$C_6$, un cyclo-alcényle en $C_5$ ou $C_6$, un alcynyle en $C_3$ ou $C_4$, éventuellement porteur d'1 ou 2 substituants pris dans l'ensemble constitué par les alkyles en $C_1$—$C_6$, le phényle, les halogènes et/ou les alcoxy en $C_1$ et $C_2$; un phényle éventuellement porteur d'1 à 3 substituants pris dans l'ensemble constitué par les alkyles en $C_1$—$C_4$, les alcoxy en $C_1$—$C_4$, les halogènes, $NO_2$ et/ou $CF_3$; un furfuryle, un tétrahydrofurfuryle ou un équivalent cationique d'une base minérale ou organique,

$R_3$ représente un alkyle en $C_1$—$C_6$, éventuellement porteur d'un alcoxy en $C_1$—$C_4$, d'un halogène ou d'un phényle, ce dernier pouvant lui-même porter de 1 à 3 substituants pris dans l'ensemble constitué par les alkyles en $C_1$—$C_4$ et les halogènes; un alcényle en $C_3$—$C_6$; ou un phényle éventuellement porteur d'1 à 3 substituants pris dans l'ensemble constitué par les alkyles en $C_1$—$C_4$ et les halogènes,

$R_4$ et $R_5$ représentent chacun, indépendamment l'un de l'autre, l'hydrogène, un alkyle en $C_1$—$C_4$, un hydroxy-alkyle en $C_1$—$C_6$, un cyclo-alkyle en $C_5$ ou $C_6$ ou un phényle, lequel porte éventuellement de 1 à 3 substituents pris dans l'ensemble constitué par les alkyles en $C_1$—$C_4$, les alcoxy en $C_1$—$C_4$, les halogénes et/ou $CF_3$ avec la restriction que $R_4$ et $R_5$ ne peuvent pas représenter en même temps chacun un phényle, ou forment ensemble une chaîne méthylénique à 2, 4 ou 5 maillons dans laquelle un groupe $—CH_2—$ peut être remplacé par O, NH ou $N(CH_3)$,

$R_6$ représente H ou $CH_3$,

$R_7$ représente H, $CH_3$ ou $C_2H_5$,

$R_8$ représente H, $CH_3$, $C_2H_5$ ou un phényle,

$R_9$ et $R_{10}$ représentent chacun, indépendamment l'un de l'autre, un alcoxy en $C_1$—$C_4$, un alkylthio en $C_1$—$C_4$, un alkylamino en $C_1$—$C_4$ ou un di-($C_1$—$C_4$)-alkylamino, ou forment ensemble un groupe de formule $=N—R_{12}$,

$R_{11}$ représente un alkyle en $C_1$—$C_6$, un halogéno-alkyle en $C_1$—$C_6$, un cyclo-alkyle en $C_3$, en $C_5$ ou en $C_6$, un alcényle en $C_3$—$C_6$, un phényle, un alkyl-phényle à alkyle en $C_1$—$C_4$, un alcoxy-phényle à alcoxy en $C_1$—$C_4$, un halogéno-phényle, un trifluorométhylphényle, un nitrophényle ou un radical de formule

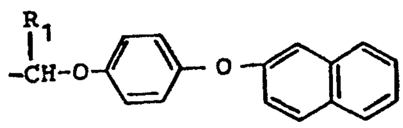

et

$R_{12}$ représente un alkyle en $C_1$—$C_4$ ou un phényle éventuellement porteur d'1 à 3 substituants pris dans l'ensemble constitué par les halogènes, $CF_3$, $NO_2$ et/ou alkyles en $C_1$—$C_4$.

2. Produits herbicides selon la revendication 1, caractérisés en ce qu'ils contiennent un composé de formule I sous la forme de son énantiomère D (le centre de chiralité étant l'atome de carbone situé entre O et $(CH_2)_q$).

3. Composés de formule I, dans lesquels $R_2$ représente un alkyle en $C_1$—$C_{12}$ éventuellement porteur d'1 à 6 substituants pris dans l'ensemble constitué par les halogènes, et/ou le groupe hydroxy, les alcoxy en $C_1$—$C_6$, les alkylthio en $C_1$—$C_4$, les ($C_1$—$C_6$)alcoxy-($C_2$—$C_6$)alcoxy, les halogénoalcoxy en $C_1$—$C_4$, le radical méthoxy-éthoxy-éthoxy, les alkylamino en $C_1$—$C_4$, les di-($C_1$—$C_4$)alkylamino, le phényle, l'oxirannyle et le phénoxy, ce dernier pouvant également porter 1 ou 2 substituants pris dans l'ensemble constitué par les halogènes et/ou les alcoxy en $C_1$—$C_4$; un cyclo-alkyle en $C_5$ ou $C_6$, un halogénocycloalkyle en $C_5$ ou $C_6$, un alcényle en $C_3$—$C_5$, un halogéno-alcényle en $C_3$—$C_6$, un cyclo-alcényle en $C_5$ ou $C_6$, un alcynyle en $C_3$ ou $C_4$, qui peut porter 1 ou 2 substituants pris dans l'ensemble constitué par les alkyles en $C_1$—$C_6$, le phényle, les halogènes et/ou les alcoxy en $C_1$ et $C_2$; un phényle éventuellement porteur d'1 ou 2 substituants pris dans l'ensemble constitué par les alkyles en $C_1$—$C_4$, les alcoxy en $C_1$—$C_4$, les halogènes, $NO_2$ et/ou $CF_3$; un furfuryle, un tétrahydrofurfuryle ou un équivalent cationique d'une base minérale ou organique, et les autres symboles ont les significations qui ont été données à la revendication 1.

4. Composés de formule I sous la forme de leurs énantiomères D (le centre de chiralité étant l'atome de carbone compris entre O et $(CH_2)_q$).

5. Composé de formule

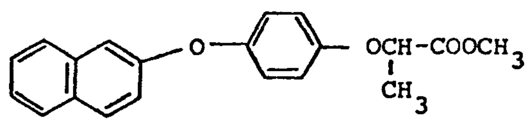

6. Composé de formule

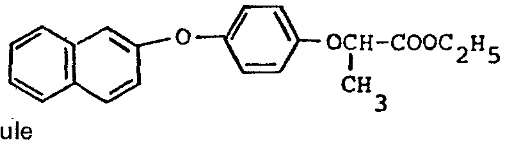

7. Composé de formule

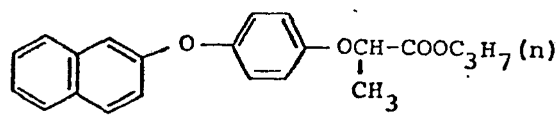

8. Composé de formule

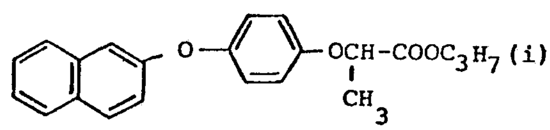

9. Procédé de préparation de composés selon la revendication 3, procédé caractérisé en ce que l'on fait réagir des ($\beta$-naphtoxy)-4 phénols de formule II

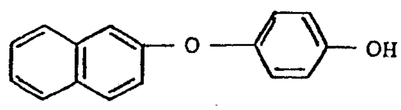

(II)

en présence d'un accepteur d'acide, ou des sels de métaux alcalins de ces phénols, avec des composés répondant à la formule III

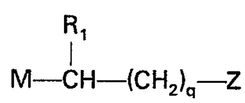

(III)

dans laquelle M représente le chlore, le brome, un groupe mésyloxy ou un groupe benzène-sulfonyloxy éventuellement substitué sur le noyau aromatique, ou à la formule IV

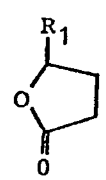

(IV)

et, si on le désire, on transforme les composés de formule I ainsi obtenus, par saponification, estérification, transestérification, salification, réduction, amidation, acylation, déshydratation, sulfhydrylation, acétalisation ou formation d'oximes, en dérivés correspondants de formule I.

10. Application de produits herbicides selon l'une des revendications 1 et 2 à la lutte contre la végétation indésirable.